# EUROPEAN PATENT APPLICATION

(11) **EP 4 018 976 A1**
(43) Date of publication of application: **29.06.2022**
(21) Application number: 20907386.5
(22) Date of filing: 09.11.2020
(51) Int. Cl.: A61F 2/95

(54) **IMPLANT CONVEYING DEVICE AND INNER TUBE ASSEMBLY THEREOF, AND CATHETER**

(30) Priority: 24.12.2019 CN 201911348433
(71) Applicant: Shanghai MicroPort CardioFlow Medtech Co., Ltd., Shanghai 201203 (CN)
(72) Inventor: WU, Xuwen, Shanghai 201203 (CN); MEI, Jie, Shanghai 201203 (CN); SHI, Ruolin, Shanghai 201203 (CN); QIU, Yao, Shanghai 201203 (CN); GUI, Baozhu, Shanghai 201203 (CN); CHEN, Guoming, Shanghai 201203 (CN); LI, Yu, Shanghai 201203 (CN)
(74) Representative: Dauncey, Mark Peter
(86) International application number: PCT/CN2020/127488
(87) International publication number: WO 2021/129195

(57) **Abstract**

Disclosed are an implant conveying device and an inner tube assembly thereof, and a catheter (100), wherein the inner tube assembly comprises an implant protection part (1), an inner tube (3) and a fixing head (2) connected to the inner tube (3), wherein the implant protection part (1) is connected to the inner tube (3) or/and the fixing head (2), the implant protection part (1) is used for being in contact with an implant to support the implant, and a protector (11, 11a, 11b) is in the shape of a circular ring sheet or a circular arc sheet. According to the provided implant conveying device, by arranging the implant protection part (1), a non-anchoring rod with weak circumferential supporting force of a valve stent (5) can be supported, and inclination of and damage to the valve stent (5) in the loading process and distortion and indentation in the releasing process are avoided.

## Description

### TECHNICAL FIELD

The present application relates to an implant conveying system, and in particular, to an implant conveying device and an inner tube assembly thereof, and a catheter.

### BACKGROUND

During loading and release, an implant, such as a self-expanding stent, is required to have a specific structure to be fixed to a corresponding structure of a conveyor by means of shape fit or cable pull, either at one end or at both ends, so as to realize stable press grip and release of the implant by the conveyor. This specific structure on the implant is called a lug, and the corresponding structure on the conveying system is called a fixing head. The lug may appear at any joint on the stent. In the case of a single-layer stent, the lug is generally arranged at one end of the sent. In the case of a double-layer stent, the lug is generally arranged at one end of the inner or outer stent. However, after the double-layer stent is pressed and gripped, the lug is not necessarily at the top. Lugs may be arranged on all joints in a circumferential direction at an end of the stent or only on some joints. Stent rods where the remaining joints without lugs are located are called non-lug rods. Depending on whether there are lugs at the joints, the stent rods with lugs may be called anchoring rods, and the stent rods without lugs may be called non-anchoring rods. In the case of the double-layer stent, the anchoring rods and the non-anchoring rods may be in different stent layers.

### SUMMARY

### Technical Problem

A conventional conveying system has the following problems. When a valve stent is a circumferentially symmetric stent, a number of joints for shape fit or cable pull on the fixing head may be less than a number of joints corresponding to the anchoring rod on the stent in most cases. Then, in the loading process, the lug is tightly integrated in a recess, and the anchoring rod is pressed and gripped by a sheathing canal/loading tool and is supported by the fixing head, while the non-anchoring rod is only pressed and gripped. Moreover, due to the existence of an axial height difference, that is, a height of the lug or a height difference formed by notches arranged on an end portion of a stent main body, the anchoring rod and the non-anchoring rod are often subjected to different forces. As a result, during the loading and release, the non-anchoring rod easily causes inward adduction, and the stent directly applies force to a valve leaflet, which easily damages the valve leaflet and affects a function of a prosthesis. Besides, uneven force of the implant causes inclination of and damage to the implant or even loading failure or unavailability of the implant. When the valve stent is designed as a stent not circumferentially symmetric, the above phenomenon is more serious. For example, in the stent, some regions are less rigid than others, or notches are asymmetrically arranged. As a result, the valve stent is subjected to uneven force and is more prone to inclination, distortion and damage during the loading and release, thereby leading to loading failure or unavailability of the implant.

A common fixing head is generally a truncated cone or wedge with a recess. The lug on the implant and the recess match in shape and are connected by shape fit. In clinical applications, an existing implant loading process involves fixing a lug into a recess of a fixing head after an implant is partially pressed and gripped, and then driving a catheter forward to gradually wrap the implant completely. In the whole loading process, especially for the circumferential asymmetrical stent, the implant is prone to inclination and damage due to poor force uniformity. Large supporting force, a short axial height and non-circumferential symmetry of the self-expanding stent may aggravate the problem of poor force uniformity of the stent. At the same time, in the releasing process, the implant may be distorted, indented, damaged or even not used normally. In addition, when a certain end face of the valve leaflet of the self-expanding stent is as high as or close to the end face of the stent, the stent may squeeze the valve leaflet during the loading and release, resulting in damage to the valve leaflet and affecting its use. In the prior art, a conveyer catheter, an implant or a loading tool is generally improved, which has a complex scheme, a long cycle and high costs, but ignores the function of the fixing head.

Therefore, there is a need to develop a new implant conveying device and an inner tube assembly thereof, which can solve the above problems.

### Technical Solution

The technical problem to be solved in the present application is to provide an implant conveying device and an inner tube assembly thereof, and a catheter, which can provide supporting force for the implant and avoid the collapse of the implant in a conveying process.

A technical solution adopted by the present application to solve the above technical problem is to provide an inner tube assembly of an implant conveying device, wherein the inner tube assembly includes an implant protection part, an inner tube and a fixing head connected to the inner tube, the implant protection part is connected to the inner tube or/and the fixing head, and the implant protection part is configured to be in contact with an implant to support the implant.

Preferably, the implant protection part includes at least one protector, and the protector is in the shape of a circular ring sheet or a circular arc sheet.

Preferably, the implant protection part is connected to the inner tube, the implant protection part includes a connector and a protector, the connector is connected to the inner tube, and the protector is connected to the connector.

Preferably, the fixing head has a recess, and the recess is arranged opposite to the protector in an axial direction.

Preferably, the implant protection part includes a first protector and a second protector connected to each other, and one of the first protector and the second protector is connected to the fixing head.

Preferably, the inner tube assembly includes a first implant protection part and a second implant protection part, the first implant protection part is connected to the fixing head, and the first implant protection part has a first protector; the second implant protection part is connected to the inner tube, the second implant protection part includes a connector and a second protector, the connector is connected to the inner tube, and the second protector is connected to the connector; and the first protector is arranged opposite to the second protector in an axial direction.

Preferably, the connector is a cylinder with an opening in the middle, and the connector is circumferentially connected to the inner tube at the opening.

Preferably, the first protector is in the shape of a circular ring sheet, and the second protector is in the shape of a circular arc sheet.

Preferably, a gap is formed between the implant protection part and the inner tube.

Another technical solution adopted by the present application to solve the above technical problem is to provide a catheter of an implant conveying device, including an outer tube and the inner tube assembly described above, the inner tube assembly passing through the outer tube.

Yet another technical solution adopted by the present application to solve the above technical problem is to provide an implant conveying device, including a handle, an outer tube and the inner tube assembly described above, the inner tube assembly passing through the outer tube, and the handle being configured to drive the outer tube to axially move relative to the inner tube assembly.

### Beneficial Effects

Compared with the prior art, the present application has the following beneficial effects. In the implant conveying device and the inner tube assembly thereof, and the catheter according to the present application, by arranging the implant protection part on the fixing head or the inner tube, regions with weak circumferential supporting force of a valve stent, such as the non-anchoring rod or notches, can be supported, and inclination of and damage to the valve stent in the loading process and distortion and indentation in the releasing process are avoided. In particular, a hollowed-out internal space is formed radially between the implant protection part and the inner tube, and the hollowed-out internal space may be used for accommodating the valve leaflet. Therefore, the implant protection part separates the valve stent from the valve leaflet, which effectively protects the valve leaflet, avoids the squeezing of the valve leaflet, effectively solves the problem of the stent squeezing the valve leaflet during the loading and release, and ensures the fatigue performance of the valve leaflet. When the protector that constitutes the implant protection part is made of a soft material, the soft material can further reduce the damage to the stent or valve while providing certain supporting force. With the development of a stent research technology, more and more stents with notches or other types of irregularities may be designed according to an original physiological structure of hearts, and the implant protection part may be used more extensively.

### BRIEF DESCRIPTION OF THE DRAWINGS

FIG. 1a is a schematic structural diagram of an implant conveying device according to an embodiment of the present application, FIG. 1b is a schematic structural diagram of an inner tube assembly of the implant conveying device according to an embodiment of the present application; and FIG. 1c is a schematic sectional view of the inner tube assembly of the implant conveying device according to an embodiment of the present application;
FIG. 2a, FIG. 2b, FIG. 2c and FIG. 2d are schematic diagrams of protectors in a variety of shapes according to an embodiment of the present application;
FIG. 3a, FIG. 3b, FIG. 3c, FIG. 3d and FIG. 3e are schematic sectional views obtained by bisecting a cross section of a catheter according to an embodiment of the present application;
FIG. 4a, FIG. 4b and FIG. 4c are schematic diagrams of distribution of the protectors according to an embodiment of the present application, among which FIG. 4b and FIG. 4c are schematic diagrams of symmetric arrangement of a plurality of protectors;
FIG. 5 is a schematic diagram of asymmetric arrangement of a plurality of protectors of the implant conveying device according to an embodiment of the present application;
FIG. 6 is a schematic structural diagram of a connection between an implant protection part and an inner tube according to an embodiment of the present application;
FIG. 7 is a schematic structural diagram of a connection between an implant protection part and a fixing head according to an embodiment of the present application;
FIG. 8 is a schematic structural diagram of a connection between an implant protection part and the inner tube and a connection between an implant protection part and the fixing head according to an embodiment of the present application;
FIG. 9 is a schematic structural diagram of a stent compressed into a sheathing canal to be in contact with the implant protection part according to an embodiment of the present application; and
FIG. 10a, FIG. 10b and FIG. 10c are schematic structural diagrams of a stent having an end with notches and a middle portion with notches and asymmetric arrangement of inflow tracts according to an embodiment of the present application.

In the drawings,
1: implant protection part, 2: fixing head, 3: inner tube, 4: conical head, 1 a: first implant protection part, 1b: second implant protection part, 11, 11a, 11b: protector, 111: first protector, 112: second protector, 12: connector, 21: lug recess, 22: base, 31: proximal inner tube, 32: distal inner tube, 5: stent, 6: sheathing canal, 7: notch, 8: lug, 901: inflow tract, 902: transition section, 903: outflow tract, 9011: special-shaped region, 51: non-anchoring rod, 52: anchoring rod, 100: catheter, 200: handle.

### DETAILED DESCRIPTION OF THE EMBODIMENTS

The present application is further described below with reference to the accompanying drawings and embodiments.

In the following description, many specific details are set forth in order to fully understand the present application. However, it is obvious for those skilled in the art that the present application may also be practiced without these specific details. Therefore, the specific details are illustrative only and may vary from the unrestrained spirit and scope and still be considered within the spirit and scope of the present application.

It is to be noted that, when one element is referred to as "fixed to" another element, it may be directly fixed to the another element or an intermediate element may exist. That is, one element is indirectly fixed to another element through the intermediate element. When one element is considered to be "connected to" another element, it may be directly connected to the another element or an intermediate element may co-exist. That is, one element is indirectly connected to another element through the intermediate element. In order to more clearly describe the structural features of the present application, "proximal", "distal" and "axial" are used as location terms. The term "proximal" means an end near an operator during surgery. The term "distal" means an end away from an operator during surgery. The term "axial" indicates a direction in which an axis of the inner tube is located. The term "or" is generally used in a sense that includes "and/or", unless otherwise expressly stated by the content.

This embodiment is described with an example in which an implant is a valve stent. In the implant conveying device and the inner tube assembly thereof, and the catheter according to the present application, an implant protection part is arranged on a fixing head and/or an inner tube, which avoids inclination of and damage to the valve stent in the loading process and distortion and indentation in the releasing process.

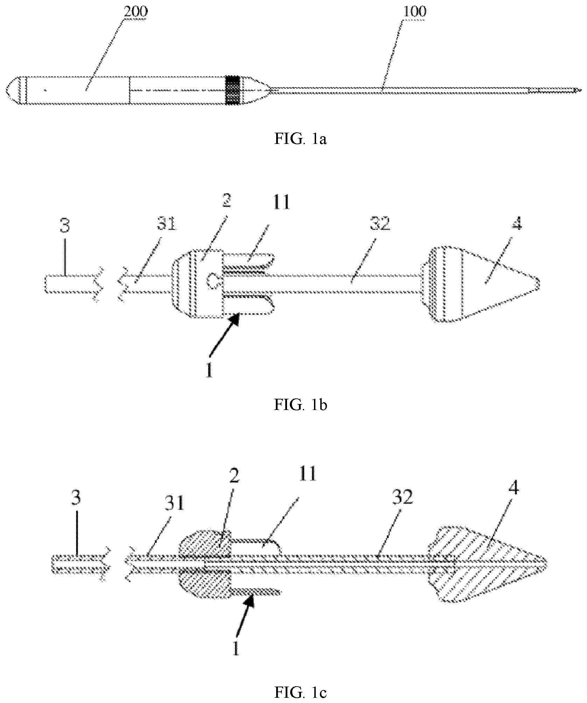

FIG. 1a is a schematic structural diagram of an implant conveying device. As shown in FIG. 1a, an implant conveying device may be applied to carrying a valve prosthesis for loading, conveying, releasing and recovering an artificial heart valve (such as an aortic artificial valve). The device may include a catheter 100 and a control handle 200 connected to a proximal end of the catheter 100. The catheter 100 may include, for example, an outer tube and inner tube assembly sequentially sheathed.

Referring to FIG. 1b and FIG. 1c, an inner tube assembly of the implant conveying device according to this embodiment includes an implant protection part 1, a fixing head 2, an inner tube 3 and a conical head 4. The fixing head 2 and the conical head 4 are connected to the inner tube 3. The inner tube 3 includes a proximal inner tube 31 and a distal inner tube 32. The proximal inner tube 31, the fixing head 2, the distal inner tube 32 and the conical head 4 sequentially connected from proximal to distal. The inner tube 3 has a penetrating inner cavity for a guidewire to pass through. In a specific embodiment, the implant protection part 1 may be connected to the fixing head 2 or fixedly connected to the inner tube 3 by integrated connection, welding, bonding, or the like, which is not specially limited in this embodiment. The implant protection part 1 is mainly configured to be in contact with a non-anchoring rod of the valve stent in a press grip state to play a supporting role. In a conveying system, one or more fixing heads 2 may be provided. It is to be understood that those skilled in the art may arrange the implant protection part 1 as required by supporting force at an end of the stent. The implant protection part 1 is optionally arranged on one fixing head, a plurality of fixing heads or an inner tube or arranged on both the fixing head and the inner tube. One or more implant protection parts 1 may be provided according to different setting positions.

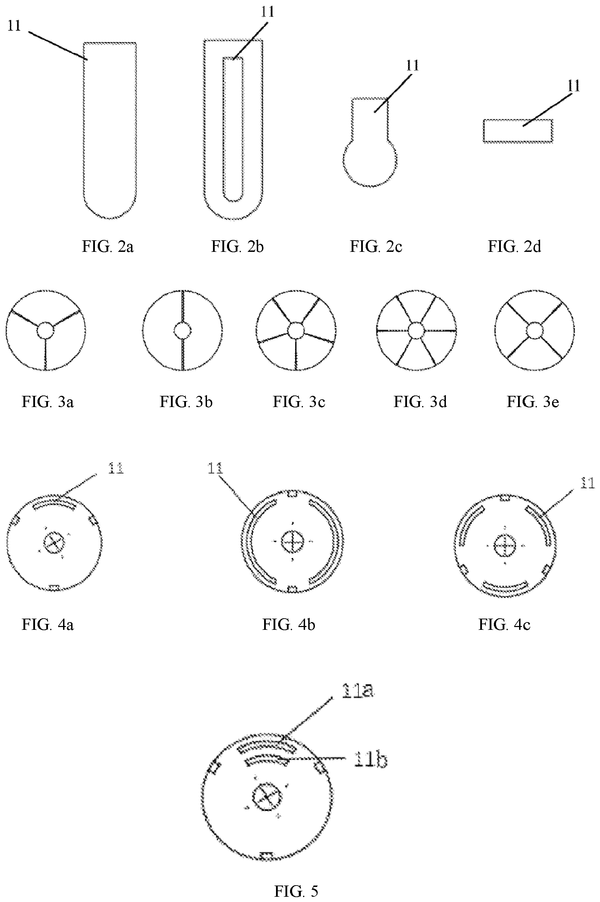

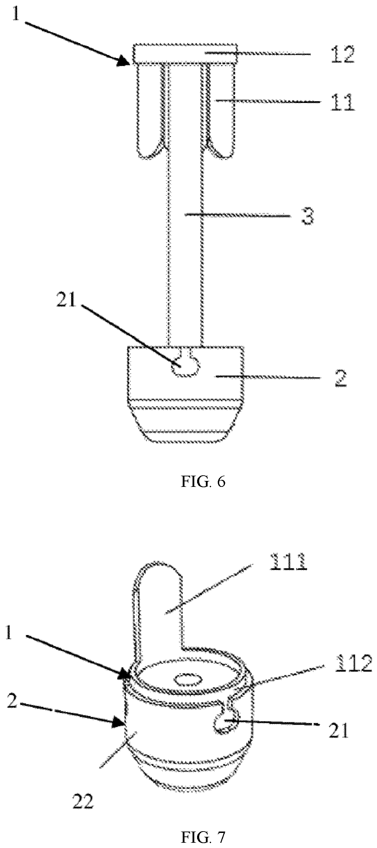

The implant protection part 1 includes at least one protector 11. In the embodiment of the present application, the protector 11 is in the shape of a circular ring sheet or a circular arc sheet. The protector 11 being in the shape of a circular ring sheet means that a projection of the protector 11 in a circumferential direction is in the shape of a circular ring sheet, for example, a second protector 112 in FIG. 7. The protector 11 being in the shape of a circular arc sheet means that the projection of the protector 11 in the circumferential direction is in the shape of a circular ring arc, as shown in FIG. 4a to FIG. 4c and FIG. 5.

The protector 11 may be in various forms. FIG. 2a to FIG. 2d show plane shapes of the protector expanded along the circumferential direction. Certainly, the protector 11 is not limited to the 4 shapes shown in FIG. 2a to FIG. 2d. Its axial length and radial width are determined according to a position and a structure of the non-anchoring rod of the valve stent to be supported, provided that the protector 11 is in contact with the non-anchoring rod at the top of the frame of the valve stent to provide supporting force and prevent the collapse of the stent. The structure is especially suitable for the loading and release of, for example, notched valve stents with incomplete symmetry at the top of the stent frame.

The arrangement of the implant protection part 1 is described as a two-dimensional plane in which the protector 11 that constitutes the implant protection unit 1 falls on a basic unit. The basic unit is a sectional view obtained by bisecting the cross section of the catheter, which includes preferably 1-8 basic units; and more preferably 2-6 basic units, as shown in FIG. 3a to FIG. 3e.

The protectors 11 may be arranged in one basic unit, as shown in FIG. 4a; or arranged in a plurality of basic units, as shown in FIG. 4b and FIG. 4c. The protectors 11 in different basic units may be the same or different in shape. When arranged in different basic units, the protectors 11 may be arranged symmetrically or asymmetrically. For example, two protectors 11 in FIG. 4b and three protectors 11 in FIG. 4c are arranged symmetrically. Asymmetric arrangement is mainly specific to asymmetric ends of a main body of the valve stent.

One basis unit may also be provided with two or more protectors 11 at the same time, which, as shown in FIG. 5, includes two protectors 11a and 11b. Certainly, the protectors 11 arranged in different basic units may be the same or different in shape.

Referring to FIG. 6, in one implementation, the implant protection part 1 is connected to the inner tube 3, and the implant protection part 1 includes a connector 12 and a protector 11. In one preferred embodiment, the connector 12 is a cylinder with an opening in the middle, and the connector 12 is circumferentially connected to the inner tube 3 at the opening and is arranged coaxially with the inner tube 3. The protector 11 is connected to the connector 12. The shape of the connector 12 is not specially limited in the present application, provided that the protector 11 can be connected to a position requiring fixed support on the stent and the size does not affect the loading and release of the stent. Preferably, a recess 21 on the fixing head 2 is arranged opposite to the protector 11 in an axial direction. That is, an opening direction of the recess 21 is opposite to an axial setting direction of the protector 11 on the connector 12. The setting may be applied to stents where the lug and the notch exist at different ends, so that the lug at one end of the stent matches the recess 21 and the notch at the other end of the stent matches and contacts the protector 11. When the lug and the notch exist at different ends of the stent, it is more likely to cause distortion or indentation of the stent. Therefore, the implant protection part 1 is arranged on the inner tube 3, so as to effectively avoid distortion or indentation of the stent by providing supporting force at the notch. When connected to the inner tube 3, one or more, preferably 1 or 2, implant protection parts 1 may be provided. The manner in which the implant protection part is arranged on the inner tube 3 is determined according to a to-be-covered position with weak circumferential supporting force of a stent, for example, a position of a non-anchoring rod, when the stent is pressed and gripped. That is, with respect to the fixing head 2, the protectors 11 may be arranged on two sides of the fixing head 2. In this case, at least two protectors 11 are required. The at least two protectors 11 may also be arranged on one side of the fixing head 2, to cover the position where the circumferential supporting force of the stent is weak. Taking a stent with a notch as an example, the protector 11 covers a non-anchoring rod where the notch is located.

In another implementation, the implant protection part 1 is directly connected to the fixing head 2, as shown in FIG. 1b, FIG. 1c and FIG. 7. The fixing head 2 includes a base 22 and a recess 21. The implant protection part 1 is connected to the base 22. The implant protection part 1 as shown in FIG. 1b and FIG. 1c includes a plurality of protectors 11 in the shape of a circular arc sheet. The implant protection part 1 as shown in FIG. 7 includes a first protector 111 and a second protector 112 connected to each other. Preferably, one or more first protectors 111 are provided and in the shape of a circular arc sheet. One second protector 112 is provided and in the shape of a circular ring shape. The second protector 112 is connected to the fixing head 2. The first protector 111 is connected to the second protector 112. The structure may be applied to loading and release of valve stents with the lug and the notch at a same end, which can provide supporting force for the notch and the non-anchoring rod at the same time and is more conducive to the uniform force of the stents.

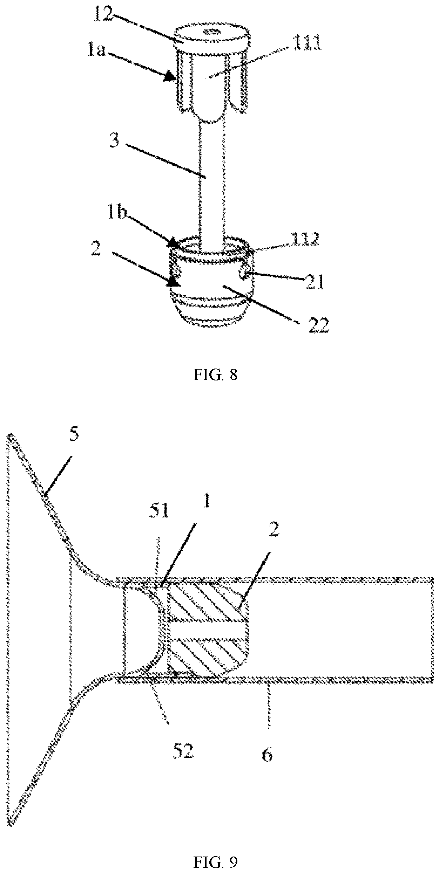

In yet another implementation, referring to FIG. 8, the inner tube assembly includes a first implant protection part 1a and a second implant protection part 1b. Preferably, the second implant protection part 1b has a second protector 112. The second protector 112 is in the shape of a circular ring sheet, and the second protector 112 is connected to the fixing head 2. The first implant protection part 1a includes a connector 12 and at least one first protector 111. The connector 12 is connected to the inner tube 3. The connector 12 is preferably a cylinder with an opening in the middle, and the connector 12 is circumferentially connected to the inner tube 3 at the opening and is arranged coaxially with the inner tube 3. The first implant protection part 1a is connected to the connector 12. The first protector 111 is in the shape of a circular arc sheet, and the first protector 111 is arranged opposite to the second protector 112 in an axial direction. That is, an axial setting direction of the first protector 111 on the connector 12 is opposite to that of the second protector 112 on the fixing head 2. The inner tube assembly of the structure may provide supporting force for the non-anchoring rods at two ends of the stent at the same time, which is more conducive to the uniform force of the stent.

In the present application, the implant protection part 1 is in contact with one end of a stent frame, so that part of the implant protection part 1 overlaps with the valve stent in the axial direction to support the valve stent. An axial length of a contact surface on which the implant protection part 1 overlaps with the stent is determined according to the position of the non-anchoring rod of the stent, to cover a length of the non-anchoring rod preferably less than 5 mm, more preferably 1-2 mm.

A material of the implant protection part 1 is not limited, which may be a hard metal material or a soft material. The soft material can further reduce the damage to the valve stent or valve while providing certain supporting force for the stent.

Preferably, the protector 11 is in the shape of a sheet and forms a hollowed-out internal space radially with the inner tube. That is, a gap is formed between the protector and the inner tube. The hollowed-out internal space may be used for accommodating a valve leaflet. The implant protection part 1 separates the valve stent from the valve leaflet, which may protect the valve leaflet, prevent squeezing of and damage to the valve leaflet, and ensure the fatigue performance of the valve leaflet. Referring to FIG. 9, the valve stent 5 includes a non-anchoring rod 51 and an anchoring rod 52. In the loading process, the stent 5, which is subjected to press grip force, may squeeze the internal valve leaflet, and the implant protection part 1 prevents inward movement of the non-anchoring rod 51. In the releasing process, in the prior art, the non-anchoring rod 51 still in the sheathing canal 6 may be deformed due to outward supporting force of a stent rod outside the sheathing canal 6, and then close up toward the interior of the stent to squeeze the valve leaflet, while the implant protection part 1 provides supporting force for the non-anchoring rod 51 in the sheathing canal 6, thereby avoiding the squeezing of the valve leaflet. When the stent is provided with a free skirt toward an axis direction of the stent, the gap formed between the implant protection part 1 and the inner tube may also be used for placing the free skirt, which avoids the squeezing of the free skirt. When the stent is a double-layer stent, the gap may also be used for placing the inner stent of the double-layer stent, thereby avoiding the squeezing of the inner stent. When the stent is loaded or released and a gap is formed between the protector and the inner tube, the protector also plays a certain cushioning role, which is more conducive to the gentle grip or release of the stent.

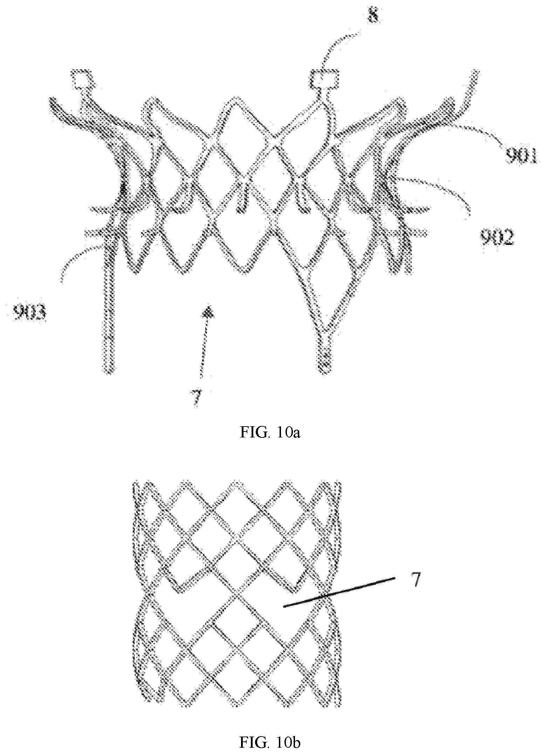

FIG. 10a shows a mitral valve replacement stent. A main structure of the stent includes a lug 8, an inflow tract 901, a transition section 902, an outflow tract 903 and a notch 7. The notch 7 and the lug 8 are arranged at two ends of the stent respectively. The stent has a short axial height and a notch. The stent has poor force uniformity in the loading process, and the stent is prone to inclination, distortion, collapse or even damage. When an implant protection part 1 is arranged at a position corresponding to the notch 7 on the inner tube assembly of the conveying system, in the loading process, a grid at the notch 7 is in contact with the implant protection part 1, which can ensure the balanced force of the outflow tract 903 of the stent and avoid the occurrence of inclination, distortion and collapse of the stent.

FIG. 10b shows another stent with a notch 7 in the middle of a main structure of the stent. An implant protection part 1 may be arranged at a position corresponding to the notch 7 on the inner tube assembly of the conveying system. The implant protection part 1 is in contact with at least part of a grid around the notch to provide supporting force, so as to ensure the balanced force of the stent and avoid the occurrence of inclination, distortion and collapse of the stent.

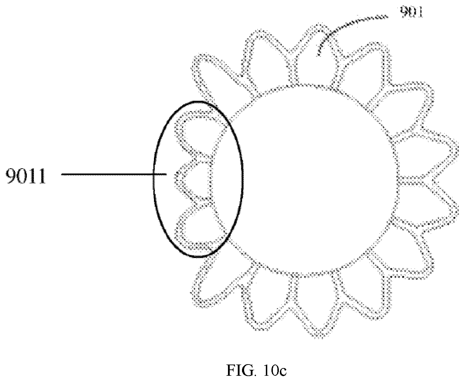

FIG. 10c shows a top view of another stent. An inflow tract 901 of the stent has a special-shaped region 9011 arranged asymmetrically. When an implant protection part 1 is arranged at a position corresponding to the special-shaped region 9011 on the inner tube assembly of the conveying system, the implant protection part 1 is in contact with the special-shaped region 9011 of the stent, which can ensure the balanced force of the inflow tract 901 of the stent and avoid the occurrence of inclination, distortion and collapse of the stent.

Therefore, in the implant conveying device according to this embodiment, the implant protection part 1 is arranged in the inner tube assembly thereof, and the protector 11 therein solves the problem of poor force uniformity caused by the large supporting force, the short axial height and asymmetry of the self-expanding stent, thereby realizing high-quality release of the artificial valve into the lesions. The present application also applies to the following situation. When the stent is a circumferential asymmetric stent, for example, in the stent, some regions are less rigid than others, or notches are asymmetrically arranged, the stent is subjected to uneven force and is more prone to inclination, distortion and damage during the loading and release, thereby leading to loading failure or unavailability of the implant. By arranging the protector on the inner tube assembly corresponding to the regions, the stent can be supported to ensure loading and release thereof. Furthermore, with the diversity of stent types, such as a split stent, each part of the stent has different requirements for supporting force, and different implant protection parts may be selected as required by each part.

Although the present application has been disclosed as above with preferred embodiments, the embodiments are not intended to limit the present application. Any person skilled in the art may make minor modifications and improvements without departing from the spirit and scope of the present application. Therefore, the protection scope of the present application shall be subject to that defined in the claims.

## Claims

1. An inner tube assembly of an implant conveying device, **characterized by** comprising an implant protection part, an inner tube and a fixing head connected to the inner tube, the implant protection part is connected to the inner tube or/and the fixing head, and the implant protection part is configured to be in contact with an implant to support the implant.

2. The inner tube assembly according to claim 1, wherein the implant protection part comprises at least one protector, and the protector is in the shape of a circular ring sheet or a circular arc sheet.

3. The inner tube assembly according to claim 1, wherein the implant protection part is connected to the inner tube, the implant protection part comprises a connector and a protector, the connector is connected to the inner tube, and the protector is connected to the connector.

4. The inner tube assembly according to claim 3, wherein the fixing head has a recess, and the recess is arranged opposite to the protector in an axial direction.

5. The inner tube assembly according to claim 1, wherein the implant protection part comprises a first protector and a second protector connected to each other, and one of the first protector and the second protector is connected to the fixing head.

6. The inner tube assembly according to claim 1, wherein the inner tube assembly comprises a first implant protection part and a second implant protection part, the first implant protection part is connected to the fixing head, and the first implant protection part has a first protector; the second implant protection part is connected to the inner tube, the second implant protection part comprises a connector and a second protector, the connector is connected to the inner tube, and the second protector is connected to the connector; and the first protector is arranged opposite to the second protector in an axial direction.

7. The inner tube assembly according to claim 3 or 6, wherein the connector is a cylinder with an opening in the middle, and the connector is circumferentially connected to the inner tube at the opening.

8. The inner tube assembly according to claim 5 or 6, wherein the first protector is in the shape of a circular ring sheet, and the second protector is in the shape of a circular arc sheet.

9. The inner tube assembly according to claim 1, wherein a gap is formed between the implant protection part and the inner tube.

10. A catheter of an implant conveying device, **characterized by** comprising an outer tube, and the inner tube assembly according to any one of claims 1-9, the inner tube assembly passing through the outer tube.

11. An implant conveying device, **characterized by** comprising a handle, an outer tube, and the inner tube assembly according to any one of claims 1-9, the inner tube assembly passing through the outer tube, and the handle being configured to drive the outer tube to axially move relative to the inner tube assembly.
